# EUROPEAN PATENT APPLICATION

(11) **EP 2 447 720 A1**
(43) Date of publication of application: **02.05.2012**
(21) Application number: 10188859.2
(22) Date of filing: 26.10.2010
(51) Int. Cl.: G01N 33/68

(54) **sFlt1 and pulmonary complications**

(71) Applicant: Roche Diagnostics GmbH, 68305 Mannheim (DE); F. Hoffmann-La Roche AG, 4070 Basel (CH)
(72) Inventor: Hess, Georg, 55130 Mainz (DE); Horsch, Andrea, 68259 Mannheim (DE); Zdunek, Dietmar, 82327 Tutzing (DE)
(74) Representative: Dick, Alexander

(57) **Abstract**

The present invention relates to the field of diagnostic measures. Specifically, it relates to a method for monitoring inflammatory pulmonary disease in a patient suffering from inflammatory pulmonary disease comprising determining the amount of the biomarker sFlt1 in a first sample and a second sample from said subject, whereby inflammatory pulmonary disease is monitored. Moreover, the present invention pertains to a method for assessing the severity of inflammatory pulmonary disease in a subject suffering from inflammatory pulmonary disease comprising determining the amount of the biomarker sFlt1 in a sample of said subject and comparing said amount to a reference amount whereby it is the severity of said inflammatory pulmonary disease is assessed. Further encompassed are diagnostic devices and kits for carrying out the aforementioned methods.

## Description

The present invention relates to the field of diagnostic measures. Specifically, it relates to a method for monitoring inflammatory pulmonary disease in a patient suffering from inflammatory pulmonary disease comprising determining the amount of the biomarker sFlt1 in a first sample and a second sample from said subject, whereby inflammatory pulmonary disease is monitored. Moreover, the present invention pertains to a method for assessing the severity of inflammatory pulmonary disease in a subject suffering from inflammatory pulmonary disease comprising determining the amount of the biomarker sFlt1 in a sample of said subject and comparing said amount to a reference amount whereby it is the severity of said inflammatory pulmonary disease is assessed. Further encompassed are diagnostic devices and kits for carrying out the aforementioned methods.

In inflammatory pulmonary disease the small bronchial and alveoli are inflamed resulting in restricted airflow in and out of the lungs. As a result, the heart has to work harder to get oxygen from the lungs. In turn, blood pressure increases as the heart works harder. Inflammatory pulmonary disease involving pathogens comprise predominantly pneumonia and chronic obstructive lung disorders (COPD).

Pneumonia may develop from upper respiratory tract infections and pneumonia is diagnosed by chest x-ray and pulmonary infiltrates (Bartlett J.G. and Mundy L.M., NEJM 1995: 333: 1618 - 24). Pneumonia may be caused by bacteria, viruses, of fungi. It may be community acquired or present as a nosocomial infection.

Pneumonia may vary with respect to its severity. Indicators for the severity of pneumonia include underlying disorders such as neoplastic disease, liver disease, congestive heart failure, cerebrovascular disease or renal failure and/or clinical findings such as low systolic blood pressure (below 90 mmHg), high pulse rate or body temperature above 40°Celsius or below 35° Celsius (Fine M.J. et al NEJM 1997, 336: 243 - 50). Specifically bacterial pneumonia may become systemic and may proceed to sepsis.

In addition pulmonary infiltrates used to diagnose pneumonia may be difficult to separate from cardiac infiltrates although radiographic features have been developed to separate between these conditions (Ely E.W. et al Chest 2002: 121: 942 - 50).

Chronic obstructive pulmonary disorders (COPD) are associated with airflow obstruction caused by hypersecretion of mucus in the airways (Fletcher C., Peto R. BMJ 1977, 1: 1645 - 8). The predominant cause of COPD is tobacco smoke, which is also a predominant risk factor for cardiovascular disorders. Superimposed bacterial infections are frequent in COPD and may be the cause of acute exacerbations, moderate to severe exacerbations can be recognized clinically by increased sputum purulence and increased sputum volume. (Sethi S., Murphy T.F., NEJM 359, 2008: 2355 - 65). As with pneumonia systemic infections may also develop in COPD.

Inflammatory lung disorders may run an uncomplicated course or may be associated with local or systemic complications. Such complications are frequently associated with local or systemic ischemia.

Soluble fms-like tyrosine kinase-1 (sFlt-1 or sVEGFR-1) is a splice variant of VEGF receptor 1 (Flt-1) which is produced by a variety of tissues. It is a tyrosine kinase protein that disables proteins that cause blood vessel growth. In patients with acute coronary syndrome it was shown, that the soluble receptor for VEGF (sFIT1) is increased. In patients with inflammatory lung disorders increased expression of FlT1 has been identified in endothelial cells and in intimal and medial vascular smooth muscle cells in patients with COPD, however sFlT1 has not been studied in these patient groups, this is however of high significance as the identification of complications of inflammatory lung disorders is so far restricted to clinical findings (Halm E.A., Teirstein A.S. NEJM 2002, 347: 2039 -2045, Ware L.B., Matthay M.A., NEJM 2005, 353: 2788 - 96, Sethi S., Murphy T.F., NEJM 2008, 359: 2355 - 2365).

Assessing the severity of inflammatory pulmonary disease and monitoring inflammatory pulmonary disease, however, is difficult. For example, Halm et al. (2002) N Engl J Med, vol 347, No. 25: 2039-2045 proposes to calculate a pneumonia severity index. However, in order to calculate the pneumonia severity index, various physical, laboratory and radiographic exams have to be carried out (determination of pulse, respiratory rate, systolic blood pressure, body temperature, arterial pH, blood urea nitrogen, sodium, glucose, hema-tocrit, pleural effusion).

Biomarkers offer diagnostic information in addition to the often unspecific results of the clinical examination of patients. Moreover, diagnostic tests based on biomarkers can often be performed at the bedside and do not require a highly skilled medical practitioner. However, the use of biomarkers in the context of pulmonary diseases has so far been restricted to the differential diagnosis of pulmonary diseases. Biomarkers for determining the severity and the course of pulmonary diseases have been lacking so far. However, such bio-marks could offer vital information to support therapeutical decisions.

The technical problem underlying the present invention can be seen as the provision of means and methods for reliably and efficiently monitoring inflammatory pulmonary disease and for assessing the severity of inflammatory pulmonary disease. The technical problem is solved by the embodiments characterized in the claims and herein below.

Accordingly, the present invention relates to a method for monitoring inflammatory pulmonary disease in a patient suffering from inflammatory pulmonary disease, said method comprises the steps of
a. determining the amount of soluble fms-like tyrosine kinase 1 (sFlt1) in a first sample of said a subject,
b. determining the amount of sFlt1 in a second sample of said subject obtained after said first sample; and
c. comparing the amount of sFlt1 in said first sample to the amount of sFlt1 in said second sample.

Preferably, said inflammatory pulmonary disease is monitored by carrying the further step of d) monitoring whether said inflammatory pulmonary disease has deteriorated or improved based on the results of the comparison carried out in c).

The method of the present invention, preferably, is an ex vivo method. Moreover, it may comprise steps in addition to those explicitly mentioned above. For example, further steps may relate to sample pre-treatments or evaluation of the results obtained by the method. The method may be carried out manually or assisted by automation. Preferably, step (a), (b) and/or (c) may in total or in part be assisted by automation, e.g., by a suitable robotic and sensory equipment for the determination in steps (a) and/or (b), a computer-implemented comparison and/or monitoring based on said comparison in step (c).

The term "subject" as used herein relates to animals, preferably mammals, and, more preferably, humans. The subject according to the present invention shall suffer from inflammatory pulmonary disease as described elsewhere herein. The subject may or may not have a history of cardiac diseases, in particular, a history of stable coronary artery disease or heart failure.

It is, however, preferred that the subject shall not have suffered from stroke, acute coronary syndrome (particularly from STEMI or NTSTEMI myocardial infarction, or from unstable angina) or from a hemorrhagic shock, preferably, within at least two weeks, at least four weeks, and, more preferably, eight weeks before the onset of symptoms of inflammatory pulmonary disease (or before the first sample is obtained from said subject).

The term "inflammatory pulmonary disease" (herein also referred to as "inflammatory lung disease"), preferably, refers to chronic obstructive pulmonary disorder (COPD) and, more preferably, acute exacerbations of COPD (thus, COPD accompanied with acute exacerbations), and, most preferably, to pneumonia.

The term "chronic obstructive pulmonary disease", abbreviated as COPD, refers to a pulmonary disorder characterized by coughing, sputum and dyspnea. COPD is a progressive disease, i.e. the severity of the symptoms usually increases with the duration of the disease. In most cases of COPD, especially in advanced cases, a cure is impossible. Therapy rather aims at slowing the progress of the disease and relief of the symptoms. In most cases, the exposure to inhaled toxins, often tobacco smoke, causes a chronic bronchitis. Chronic bronchitis leads to increased secretion of mucus, swelling of the mucosa and bronchospasm. The swelling associated with inflammation combined with the increased metabolic activity of the affected tissue lead to ischemia at the site of inflammation. The airflow in the lungs is, thus, obstructed. One consequence of inflammation is the thickening of the walls of the airways. This thickening is mediated - amongst other mechanisms - by changes in the airway mucosal blood vessels. This process is promoted by pro-angiogenic factors, preferably vascular endothelial growth factor (VEGF), transforming growth factor β (TGF β) and fibroblast growth factors (FGFs) (Zanini et al., 2008, Therapeutic Perspectives in Bronchial Vascular remodelling in COPD, Therapeutic Advances in Respiratory Disease, 2: 179-187). Most cases of COPD are caused by tobacco smoke. Occupational exposition to dust, isocyanates and fume from welding are also important causes of COPD. In non-smokers COPD may also be caused by a α1-antitrypsin deficiency.

In most cases COPD does not progress steadily, but rather shows periods of stable symptoms interrupted by periods of a sudden worsening of the disease, the acute exacerbations.

If the inflammatory pulmonary disease is COPD, the COPD is, preferably, accompanied by acute exacerbations. Thus, the subject with COPD shall, preferably, suffer from acute exacerbations.

Exacerbations are characterized by an increase of sputum and/or increased dyspnea. Acute exacerbations are triggered by bacterial or viral infection of the airways or by environmental pollutants. Inflammation is increased during acute exacerbations. An acute exacerbation of COPD typically lasts for several days. Airway inflammation is increased during the exacerbation resulting in increased hyperinflation, reduced expiratory air flow and worsening of gas transfer.

Moreover, the subject suffering from COPD shall have a FEV1[%] of, preferably, lower than 50%, more preferably, lower than 40% and most preferably, of lower than 30%. FEV1[%] is the forced expiratory volume in 1 second, i.e. the relative amount of air which can be forcibly exhaled from the lungs in the first second of a forced exhalation. It can be determined by well known methods, preferably, by spirometry (see Rabe et al. (2007) Global Strategy for the Diagnosis, Management, and Prevention of Chronic Obstructive Pulmonary Disease: GOLD Executive Summary" Am. J. Respir. Crit. Care Med. 176 (6): 532-55).

The term "pneumonia" is understood by the skilled person. As used herein, the term, preferably, refers to an acute infection of one or both lungs. Pneumonia may be caused by an infection of the lung by bacteria, fungi or viruses. In the context of the method of the present invention, pneumonia is preferably caused by bacteria. Bacteria which cause pneumonia include Streptococcus pneumoniae, Staphylococcus aureus, Haemophilus influenzae, Klebsiella pneumoniae, Escherichia coli, Pseudomonas aeruginosa, and Moraxella cata-rrhalis. Chest pain, dyspnea and fever are other frequently found symptoms. Pneumonia is either primary or secondary pneumonia. Primary pneumonia is, preferably, not precipitated by other pre-existing diseases or disorders. Secondary pneumonia is the complication of another pre-existing disease or disorder. Several diseases increase the risk of pneumonia in a patient. Diseases promoting pneumonia are, preferably, pulmonary oedema (caused by heart failure). Pneumonia may also be promoted by a suppressed immune system (e.g., in HIV or cancer patients, or in patients suffering from an autoimmune disease).

Generally, the prognosis of patients with secondary pneumonia is worse than that of patients with primary pneumonia. A secondary pneumonia typically requires hospitalization of the patient while a primary pneumonia may in many cases be treated at home. In the context of the method of the present invention, it is particularly contemplated that the subject suffers from secondary pneumonia.

The severity of pneumonia can be assessed by determining the pneumonia severity index (PSI). When applying this index, subjects are allocated in five risk class groups, i.e. risk class groups I to V. Whereas patients suffering from risk class IV or V pneumonia have a significantly increased risk of mortality, patients with risk class I, II or III have a low or moderate risk of mortality. How to determine the risk class according to the PSI is described elsewhere herein. In the context of the method of the present invention, the subject shall preferably suffer from risk class I, II, III, or, more preferably, from risk class IV or from risk class V pneumonia.

The term "monitoring" as used herein, preferably, refers to assessing the course of inflammatory pulmonary disease. Preferably, by assessing the course of inflammatory pulmonary disease, it can be determined whether the condition of a subject suffering from inflammatory pulmonary disease improves or deteriorates. As will be understood by those skilled in the art, such an assessment is usually not intended to be correct for 100% of the subjects to be monitored. The term, however, requires that the assessment is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

The "first sample" can obtained at any time after the onset of symptoms of inflammatory pulmonary disease. Preferably, the first sample is obtained 1 day, 2 days, 3 days, 4 days, 5 days or one week after the onset of symptoms of inflammatory pulmonary disease. Of course, the first sample shall be obtained before obtaining the second sample.

The "second sample" is, preferably, understood as a sample which is obtained in order to reflect a change of the amount of the respective marker as compared to the amount of the respective marker in the first sample. The second sample shall be obtained after the first sample. Preferably, the second sample is not obtained too early after the first sample (in order to observe a sufficiently significant change to allow for monitoring). Thus, the "second sample" is preferably obtained within 12 to 96 hours, more preferably within 24 to 72 hours, 24 to 48 hours. It is particularly contemplated to obtain said second sample preferably, 96 hours, 72 hours, 48 hours or 24 hours after said first sample.

Preferably, at least one further sample is obtained in order to further monitor the change of the amount of a biomarker referred to herein. Such further sample may be obtained, preferably, within 24 to 72 hours, and more preferably 18 to 48 hours after the second sample.

The term "soluble Flt-1" or "sFlt-1" as used herein refers to polypeptide which is a soluble form of the VEGF receptor Flt1. It was identified in conditioned culture medium of human umbilical vein endothelial cells. The endogenous soluble Flt1 (sFlt1) receptor is chromatographically and immunologically similar to recombinant human sFlt1 and binds [1251] VEGF with a comparable high affinity. Human sFlt1 is shown to form a VEGF-stabilized complex with the extracellular domain of KDR/Flk-1 in vitro. Preferably, sFlt1 refers to human sFlt1. More preferably, human sFlt1 can be deduced from the amino acid sequence of Fit-1 as shown in Genbank accession number P17948, GI: 125361. An amino acid sequence for mouse sFlt1 is shown in Genbank accession number BAA24499.1, GI: 2809071.

The term "sFlt-1" used herein also encompasses variants of the aforementioned specific sFlt-1 polypeptide. Such variants have at least the same essential biological and immunological properties as the specific sFlt-1 polypeptide. In particular, they share the same essential biological and immunological properties if they are detectable by the same specific assays referred to in this specification, e.g., by ELISA assays using polyclonal or monoclonal antibodies specifically recognizing the said sFlt-1 polypeptide. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific sFlt-1 polypeptide (preferably over the whole length of said polypeptide. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments or subunits of the specific sFlt-1 polypeptide or the aforementioned types of variants as long as these fragments have the essential immunological and biological properties as referred to above. Such fragments may be, e.g., degradation products of the sFlt-1 peptide. Further included are variants which differ due to posttranslational modifications such as phosphorylation or myristylation.

Determining the amount of sFlt-1 or any other peptide or polypeptide referred to in this specification (e.g. of a natriuretic peptide or of a cardiac troponin) relates to measuring the amount or concentration, preferably semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal - may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labelled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immuno-assays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Suitable methods are described in the following.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labeled with a detectable label prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand. Labelling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labelled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of a marker as referred to herein in a first sample with an amount of said marker in a second sample. The terms "first sample" and "second sample" are specified elsewhere in this description. It is to be understood that comparing as used herein refers to a comparison of corresponding parameters or values. It is to be understood that an amount of a marker is a first sample is compared to an amount of the respective marker, thus the same marker, in a second sample. The comparison referred to in the context of the method of the present invention may be carried out manually or computer assisted. For a computer assisted comparison, the value of the determined amount in the second sample may be compared to a value of the determined amount in a first sample which is stored in a database by a computer program. The computer program may further evaluate the result of the comparison, i.e. automatically provide the desired assessment in a suitable output format. Based on the comparison of the amount of a biomarker as referred to herein in a first sample to the amount in a second sample it is possible to monitor inflammatory pulmonary disease.

As set forth above, the monitoring is, preferably, based on the comparison of the amount of a sFlt1 of the present invention in a first sample to the amount of the sFlt1 in a second sample obtained after said first sample.

Preferably, an increase, and more preferably a statistically significant increase of the amount sFlt1 in said second sample compared to the amount in said first sample indicates deterioration of inflammatory pulmonary disease, and, thus, that the inflammatory pulmonary disease has deteriorated during the period between obtaining the first and the second sample.

Preferably, a decrease of the amount in said second sample as compared to said first sample indicates amelioration of said inflammatory pulmonary disease, and, thus, that the inflammatory pulmonary disease has improved during the period between obtaining the first and the second sample.

Particularly, a significant increase (or decrease) is an increase (or decrease) of a size which is considered to be significant for, particularly statistically significant. The terms "significant" and "statistically significant" are known to the person skilled in the art. Whether a change or an increase (or decrease) is statistically significant can be determined without further ado by the person skilled in the art using various well known statistic evaluation tools including those referred to herein.

Moreover, an increase of the amount in the second sample as compared to the amount in the first sample which is considered to be significant is an increase of the amount in the second sample as compared to the first sample in a subject or a group of subjects in which the inflammatory pulmonary disease has deteriorated. Moreover, a decrease of the amount in the second sample as compared to the amount in the first sample which is considered to be significant is a decrease of the amount in the second sample as compared to the first sample in a subject or a group of subjects in which the inflammatory pulmonary disease has improved.

Moreover, an unchanged amount, preferably an essentially unchanged amount, in said second sample as compared to said first sample, preferably, indicates no change in status of said inflammatory pulmonary disease.

Preferred significant increases of the amounts of sFlt1 which have been found in the course of the invention which indicate that inflammatory pulmonary disease has deteriorated are given below.

According to the invention, an increase of the amount of sFlt1 in the second sample compared to the amount in the first sample, preferably, of at least 10% more preferably of at least 30 % and even, more preferably, of at least 50 %, of at least 100% and most preferably of at least 200% is considered to be significant and, thus, to be indicative for a deterioration of inflammatory pulmonary disease.

Moreover, an increase of the amount of sFlt1 in the second sample compared to the amount in the first sample, preferably, of at least 50 pg/ml, more preferably of at least 100 pg/ml and even, more preferably, of at least 150 pg/ml, and most preferably of at least 300 pg/ml or 500 pg/ml is considered to be significant and, thus, to be indicative for a deterioration of inflammatory pulmonary disease.

Preferably, a decrease of the amount of sFlt1 in the second sample compared to the amount in the first sample, preferably, of at least 10% more preferably of at least 20 % and even, more preferably, of at least 30%, and most preferably of at least 50 % or 75% is considered to be significant and, thus, to be indicative for an amelioration/improvement of inflammatory pulmonary disease.

Moreover, a decrease of the amount of sFlt1 in the second sample compared to the amount in the first sample, preferably, of at least 50 pg/ml, more preferably of at least 100 pg/ml and even, more preferably, of at least 150 pg/ml, and most preferably of at least 300 pg/ml or 500 pg/ml is considered to be significant and, thus, to be indicative for an amelioration/improvement of inflammatory pulmonary disease.

Advantageously, it has been found in the studies underlying the present invention that i) the determination of the amount of sFlt1 in a first sample and in a second sample of a subject suffering from inflammatory pulmonary disease and ii) the comparison of the amount of sFlt1 in said first sample to the amount in said second sample allows for reliably monitoring inflammatory pulmonary disease. It was found that an increase of sFlt1 in a second sample as compared to a first (earlier obtained) sample indicates deterioration of inflammatory pulmonary disease, whereas a decrease of sFlt1 in a second sample as compared to a first (earlier obtained) sample indicates amelioration of inflammatory pulmonary disease (see accompanying Examples, below).

Thanks to the present invention, it is possible to more reliably monitor inflammatory pulmonary disease, in particular in emergency situation where a fast and reliable monitoring is required. Moreover, the time consuming, expensive and cumbersome diagnostic measures such as scoring systems can be avoided when applying the method of the invention as an aid for diagnosis.

In a preferred embodiment, the method further comprises the determination of the amount of a natriuretic peptide and/or a cardiac Troponin in said first sample and said second sample of said subject, and comparing the amount of said natriuretic peptide and/or said cardiac Troponin in said first sample to the amount of said natriuretic peptide and/or said cardiac Troponin in said second sample.

The additional determination of a natriuretic peptide and/or of a cardiac troponin in a first and a second sample from a subject suffering from inflammatory pulmonary disease allows for monitoring and assessing the cardiac condition, in particular heart failure, in said subject. Particularly, it allows for assessing whether the cardiac condition has improved or deteriorated between obtaining the first and second sample.

As used herein, the term "cardiac condition", preferably, relates to the pathological condition of the heart or blood vessel. More preferably, it relates to the function of the heart, in particular the pumping of blood in systemic and pulmonary circulation. Most preferably, the term relates to the function of the right heart. By monitoring the cardiac condition, it can be, preferably, assessed whether a subject suffering from inflammatory pulmonary disease suffers from a cardiac event or is at risk of suffering thereof (preferably, within 1 day, 2 days, 3 days or one week after the second sample is obtained). A subject, whose cardiac condition has deteriorated, may suffer from a cardiac event or may be at risk thereof. A cardiac event in the context of the present invention, preferably, is selected from the group consisting of ACS (acute coronary syndrome), heart failure and arrhythmia. It is particularly contemplated that the cardiac event is heart failure, in particular right heart failure.

In case of heart failure, the subject may have already exhibited heart failure at the onset of inflammatory pulmonary disease and the heart failure may have worsened as a consequence of inflammatory pulmonary disease (worsened heart failure). Moreover, the subject may not have exhibited heart failure at the onset of inflammatory pulmonary disease. However, said subject may have developed heart failure as a consequence of inflammatory pulmonary disease (new heart failure).

The term "natriuretic peptide" comprises Atrial Natriuretic Peptide (ANP)-type and Brain Natriuretic Peptide (BNP)-type peptides and variants thereof having the same diagnostic potential (see e.g. Bonow, 1996, Circulation 93: 1946-1950). ANP-type peptides comprise pre-proANP, proANP, NT-proANP, and ANP. BNP-type peptides comprise pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP). Preferred natriuretic peptides according to the present invention are NT-proANP, ANP, NT-proBNP, BNP, and variants thereof. ANP and BNP are the active hormones and have a shorter half-life than their respective inactive counterparts, NT-proANP and NT-proBNP. BNP is metabolised in the blood, whereas NT-proBNP circulates in the blood as an intact molecule and as such is eliminated renally. The in-vivo half-life of NTproBNP is 120 min longer than that of BNP, which is 20 min (Smith 2000, J Endocrinol. 167: 239-46.). Preanalytics are more robust with NT-proBNP allowing easy transportation of the sample to a central laboratory (Mueller 2004, Clin Chem Lab Med 42: 942-4.). Blood samples can be stored at room temperature for several days or may be mailed or shipped without recovery loss. In contrast, storage of BNP for 48 hours at room temperature or at 4° Celsius leads to a concentration loss of at least 20 % (Mueller loc.cit.; Wu 2004, Clin Chem 50: 867-73.). Therefore, depending on the time-course or properties of interest, either measurement of the active or the inactive forms of the natriuretic peptide can be advantageous. The most preferred natriuretic peptides according to the present invention are NT-proBNP and variants thereof. As briefly discussed above, the human NT-proBNP, as referred to in accordance with the present invention, is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913 or Bonow loc. cit. Preferably, human NT-proBNP as used herein is human NT-proBNP as disclosed in EP 0 648 228 B1. These prior art documents are herewith incorporated by reference with respect to the specific sequences of NT-proBNP and variants thereof disclosed therein. The NT-proBNP referred to in accordance with the present invention further encompasses allelic and other variants of said specific sequence for human NT-proBNP discussed above. Specifically, envisaged are variant polypeptides which are on the amino acid level at least 60 % identical, more preferably at least 70 %, at least 80 %, at least 90 %, at least 95 %, at least 98% or at least 99 % identical, to human NT-proBNP. The degree of identity between two amino acid sequences, in principle, can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith 1981, Add. APL. Math. 2:482, by the homology alignment algorithm of Needleman 1970, J. Mol. Biol. 48:443, by the search for similarity method of Pearson 1988, Proc. Natl. Acad Sci. (USA) 85: 2444, by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human NT-proBNP as long as the said polypeptides have NT-proBNP properties. NT-proBNP properties as referred to herein are immunological and/or biological properties. Preferably, the NT-proBNP variants have immunological properties (i.e. epitope composition) comparable to those of NT-proBNP. Thus, the variants shall be recognizable by the aforementioned means or ligands used for determination of the amount of the natriuretic peptides. Biological and/or immunological NT-proBNP properties can be detected by the assay described in Karl et al. (Karl 1999, Scand J Clin Invest 59:177-181), Yeo et al. (Yeo 2003, Clinica Chimica Acta 338:107-115). Variants also include posttranslationally modified peptides such as glycosylated or myristylated peptides. Further, a variant in accordance with the present invention is also a peptide or polypeptide which has been modified after collection of the sample, for example by covalent or non-covalent attachment of a label, particularly a radioactive or fluorescent label, to the peptide.

The term "cardiac troponin" refers to all troponin isoforms expressed in cells of the heart and, preferably, the subendocardial cells. These isoforms are well characterized in the art as described, e.g., in Anderson 1995, Circulation Research, vol. 76, no. 4: 681-686 and Ferrieres 1998, Clinical Chemistry, 44: 487-493. Preferably, cardiac troponin refers to troponin T and/or troponin I, and, most preferably, to troponin T. It is to be understood that isoforms of troponins may be determined in the method of the present invention together, i.e. simultaneously or sequentially, or individually, i.e. without determining the other isoform at all. Amino acid sequences for human troponin T and human troponin I are disclosed in Anderson, loc cit and Ferrieres 1998, Clinical Chemistry, 44: 487-493. The term "cardiac troponin" encompasses also variants of the aforementioned specific troponins, i.e., preferably, of tropoinin T or troponin I. Such variants have at least the same essential biological and immunological properties as the specific cardiac troponins. In particular, they share the same essential biological and immunological properties if they are detectable by the same specific assays referred to in this specification, e.g., by ELISA Assays using polyclonal or monoclonal antibodies specifically recognizing the said cardiac troponins. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific troponin (preferably, over the whole range). Variants may be allelic variants or any other species specific homologs, paralogs, or orthologs. Moreover, the variants referred to herein include fragments of the specific cardiac troponins or the aforementioned types of variants as long as these fragments have the essential immunological and biological properties as referred to above. Such fragments may be, e.g., degradation products of the troponins. Further included are variants which differ due to posttranslational modifications such as phosphorylation or myristylation.

Preferably, a decrease of the amount of a natriuretic peptide and/or of a cardiac Troponin in the second sample compared to the amount in the first sample, preferably, of at least 10% more preferably of at least 20 % and even, more preferably, of at least 30%, and most preferably of at least 50 % or 75% is considered to be significant and, thus, to be indicative for an amelioration/improvement of the cardiac condition of said subj ect.

Moreover, a decrease of the amount of a natriuretic peptide, in particular of NT-proBNP, in the second sample compared to the amount in the first sample, preferably, of at least 50 pg/ml, more preferably of at least 100 pg/ml and even, more preferably, of at least 150 pg/ml, and most preferably of at least 300 % or 500 pg/ml is considered to be significant and, thus, to be indicative for amelioration/improvement of the cardiac condition of said subject.

Advantageously, it has been shown that the determination of a cardiac troponin and/or a natriuretic peptide in a first and a second sample of a subject, and comparing the amount of said cardiac troponin and/or said natriuretic peptide in said sample to the amount in the first sample allows for a further monitoring of a subject suffering from inflammatory pulmonary disease. Particularly, it has been found, that it allows for assessing whether the cardiac condition, in particular the function of the right heart, has improved or deteriorated. The assessment of the cardiac condition in a subject suffering from inflammatory pulmonary disease, in particular in a subject suffering from bacterial pneumonia, is of importance since a large portion of subjects hospitalized with inflammatory pulmonary disease had one or more major cardiac event at admission (or after admission), see e.g. Musher et al. The association between pneumococcal pneumonia and acute cardiac events. Clin Infect Dis 2007; 45:158-65. Since subjects which concurrently suffer from a inflammatory pulmonary disease and a cardiac event have a significantly higher mortality than those with pneumonia alone, it is important to identify a subject who also suffers from a cardiac event or who is at risk of suffering thereof. Once a subject is suffers from a cardiac event or who is at risk of suffering thereof is identified, immediate actions can be taken.

In a preferred embodiment of the aforementioned method, the method further comprises recommending a therapy for inflammatory pulmonary disease.

The term "recommending" as used herein means establishing a proposal for a therapy which could be applied to the subject. However, it is to be understood that applying the actual therapy whatsoever is not comprised by the term. The therapy to be recommended depends on the outcome of the diagnosis provided by the method of the present invention.

Preferably, the therapy for inflammatory pulmonary disease is oral or intravenous administration of antibiotics, respiratory therapy, or administration of loop diuretics.

Preferably, if the amount of sFlt1 in the second sample as compared to the first sample is increased (and, thus, if the inflammatory pulmonary disease has deteriorated), the therapy for inflammatory pulmonary disease, in particular for bacterial pneumonia, is intravenous administration of antibiotics. Moreover, a further therapy for inflammatory pulmonary disease is respiratory therapy if the amount of sFlt1 in the second sample as compared to the first sample is increased is, particularly if the pO₂ level is low. Further preferred therapies for inflammatory pulmonary disease, particularly for COPD, are administration of bronchodilators such as short-acting β2-agonists, long-acting β2-agonists (preferably, Salmeterol, Formoterol Bambuterol or Indacaterol), or anticholinergics (preferably, ipratropium bromide, oxitropium bromide or tiotropium), oral or intravenous administration of steroids (preferably, prednisone), or administration of theophylline. In addition, if the amount of a cardiac troponin and/or a natriuretic peptide in the second sample as compared to the first sample is increased (and, thus, if the cardiac condition has deteriorated), the therapy for inflammatory pulmonary disease, preferably, further comprises the administration of loop diuretics. Moreover, the subject is, preferably, hospitalized.

Preferably, if the amount of sFlt1 in the second sample as compared to the first sample is decreased (and, thus, if the inflammatory pulmonary disease has improved), the therapy for inflammatory pulmonary disease (and in particular for bacterial pneumonia) is oral administration of antibiotics. Moreover, the subject whose inflammatory pulmonary disease has improved, preferably, shall not be hospitalized or - in case the subject was hospitalized when the first and/or second sample was obtained - may be discharged from hospital.

Antibiotics for the treatment of inflammatory pulmonary disease, and in particular for the treatment of bacterial pneumonia are well known in the art. It is to be understood that the choice of antibiotics may depend on the bacterial strain that caused the inflammation. Moreover, with respect to pneumonia the choice may depend on whether the disease is community acquired or hospital acquired. Preferred antibiotics are cefuroxime, ampicillin-clavulanate, ofloxacin, trimethoprim-sulfanethoxazole, and erythromycin.

The term "intravenous administration" refers to administration into the vein of a subject.

The term "oral administration" refers to administration via the mouth, preferably, through ingestion.

As used herein, the term "loop diuretics" pertains to diuretics that act on the ascending loop of Henle in the kidney. Loop diuretics are well known in the art. Preferred loop diuretics in the context of the method of the present invention are furosemide, bumetanide, ethacrynic acid and torsemide.

It is to be understood that the definitions and explanations of the terms made above and below apply accordingly for all embodiments described in this specification and the accompanying claims. Moreover, they apply mutatis mutandis to the following (except stated otherwise).

Moreover, the present invention relates to method for diagnosing the severity of inflammatory pulmonary disease in a subject suffering from inflammatory pulmonary disease, said method comprising the steps of
a. determining the amount of soluble fms-like tyrosine kinase 1 (sFlt1) in a sample of said a subject,
b. comparing said amount of sFlt1 to a reference amount, whereby the severity of inflammatory pulmonary disease is diagnosed.

Preferably, the diagnosis is based on the result of the comparison carried out in step b).

The aforementioned method, preferably, is an ex vivo method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b).

The term "diagnosing" as used herein means assessing whether a subject suffering from inflammatory pulmonary disease suffers from a mild or severe form of inflammatory pulmonary disease. Thus, by carrying out the claimed method, it can be differentiated in a between a mild or severe form of inflammatory pulmonary disease. As will be understood by those skilled in the art, such an assessment is usually not intended to be correct for 100% of the subjects to be diagnosed. The term, however, requires that the assessment is correct for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

A subject suffering from a mild form of inflammatory pulmonary disease, preferably, has a low risk of mortality caused by said inflammatory pulmonary disease. In the context of the method of the present invention, a risk of mortality of below 3% or 2%, and more, preferably, of below 1% (preferably, within a predictive window of 30 days) is considered as low risk of mortality.

A subject suffering from a severe form of inflammatory pulmonary disease, preferably, has a high risk of mortality caused by said inflammatory pulmonary disease. In the context of the method of the present invention, a risk of mortality of larger than 3 % or 5%, larger than 10 %, and more, preferably, larger than 15% (preferably, within a predictive window of 30 days) is considered as high risk of mortality.

With respect to pneumonia, a subject suffering from a mild form of pneumonia, preferably, has Risk Class I, II, or III pneumonia according to the Pneumonia Severity Index (PSI). Moreover, a subject suffering from a severe form of pneumonia has Risk Class IV or, more preferably, Risk Class V pneumonia according to the Pneumonia Severity Index (PSI).

The pneumonia severity index (also referred to as PORT Score) is a clinical prediction rule that medical practitioners can use to calculate the probability of morbidity and mortality among patients pneumonia. How to determine PSI is well known in the art. Preferably, the PSI is determined as decribed by Fine MJ, Auble TE, Yealy DM, Hanusa BH, Weissfeld LA, Singer DE, Coley CM, Marrie TJ, Kapoor WN. A prediction rule to identify low-risk patients with community-acquired pneumonia. N Engl J Med. 1997 Jan 23;336(4):243-250 or by Halm et al. (2002) N Engl J Med, vol 347, No. 25: 2039-2045 which are herewith incorporated by reference with respect to the entire disclosure content.

With respect to COPD, a subject suffering from a mild form of COPD, preferably, has a FEV1[%] of larger than 50%, 60%, 70%, or, more preferably, of larger than 80%.. Moreover, a subject suffering from a severe form of COPD, preferably, has a FEV1 [%] of lower than 50%, more preferably, of lower than 40%, and most preferably of lower than 30%.

In a preferred embodiment of the present invention, the subject has a history of heart failure. Thus, the subject shall have suffered from heart failure before the onset of inflammatory pulmonary disease. It is well known that subject with a history of heart failure are at increased risk of mortality when suffering from inflammatory pulmonary disease.

The term "heart failure" as used herein relates to an impaired systolic and/or diastolic function of the heart. Preferably, heart failure referred to herein is also chronic heart failure. Heart failure can be classified into a functional classification system according to the New York Heart Association (NYHA). Patients of NYHA Class I have no obvious symptoms of cardiovascular disease but already have objective evidence of functional impairment. Physical activity is not limited, and ordinary physical activity does not cause undue fatigue, palpitation, or dyspnea (shortness of breath). Patients of NYHA class II have slight limitation of physical activity. They are comfortable at rest, but ordinary physical activity results in fatigue, palpitation, or dyspnea. Patients of NYHA class III show a marked limitation of physical activity. They are comfortable at rest, but less than ordinary activity causes fatigue, palpitation, or dyspnea. Patients of NYHA class IV are unable to carry out any physical activity without discomfort. They show symptoms of cardiac insufficiency at rest. Heart failure, i.e., an impaired systolic and/or diastolic function of the heart, can be determined also by, for example, echocardiography, angiography, szintigraphy, or magnetic resonance imaging. This functional impairment can be accompanied by symptoms of heart failure as outlined above (NYHA class II-IV), although some patients may present without significant symptoms (NYHA I). Moreover, heart failure is also apparent by a reduced left ventricular ejection fraction (LVEF). More preferably, heart failure as used herein is accompanied by a left ventricular ejection fraction (LVEF) of less than 60%, of 40% to 60% or of less than 40%.

Whether the subject has a history of heart failure can be determined, e.g., by assessing the medical records of said patients. Moreover, it can be determined if a sample from said subject obtained before the onset of inflammatory pulmonary disease is available. Preferably, said sample was obtained not more than 12 months, not more than 6 months, not more than 3 months, and, more preferably, not more than 1 month before the onset of inflammatory pulmonary disease. Preferably, an amount of a natriuretic peptide, in particular of NT-proBNP, in said sample larger than 125 pg/ml, and more preferably, of more than 250 pg/ml, and, most preferably, larger than 500 pg/ml in a sample obtained before the onset of symptoms indicates that the subject suffering from inflammatory pulmonary disease has a history of heart failure and, thus, that said subject suffered from heart failure before the onset of inflammatory pulmonary disease. Preferably, an amount of a natriuretic peptide, in particular of NT-proBNP, in said sample lower than 125 pg/ml, and more preferably, of lower than 90 pg/ml, indicates that the subject suffering from inflammatory pulmonary disease has no history of heart failure and, thus, that said subject did not suffer from heart failure before the onset of inflammatory pulmonary disease.

The term "comparing" as used in the context with the aforementioned method encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, it is possible to assess whether the subject suffers from a mild form or severe form of inflammatory pulmonary disease. Therefore, the reference amount is to be chosen so that either a difference or a similarity in the compared amounts allows identifying those test subjects which belong into the group of subjects suffering from a severe or mild form of inflammatory pulmonary disease. The method allows either excluding (rule-out) or identifying (rule-in) subject as a subject suffering from (i) a mild form of inflammatory pulmonary disease or (ii) a severe form of inflammatory pulmonary disease.

The term "reference amount" as used herein refers to an amount which allows for allocation of a subject into either the group of subjects suffering from a mild form of inflammatory pulmonary disease or the group of subjects suffering from a severe form of inflammatory pulmonary disease. Such a reference amount can be a threshold amount which separates these groups from each other. Accordingly, the reference amount for a biomarker as referred to herein shall be an amount which allows for allocation of a subject into a group of subjects suffering from a mild form of inflammatory pulmonary disease or into a group of subjects suffering from a severe form of inflammatory pulmonary disease. A suitable threshold amount separating the two groups can be calculated without further ado by the statistical tests referred to herein elsewhere based on amounts of a biomarker, particularly sFlt1, from either a subject or group of subjects known to suffer from a mild form of inflammatory pulmonary disease or a subject or group of subjects known to suffer from a severe form of inflammatory pulmonary disease. Moreover, the subject or group of subjects may have or may not have a history of heart failure. Also, the subject or group of subjects may or may not suffer from heart failure caused by inflammatory pulmonary disease.

Preferably, said reference amount is derived from a subject or a group of subjects known to suffer from a severe form of inflammatory pulmonary disease and wherein an essentially identical amount or an increased amount for the biomarker, particularly sFlt1, in the sample of the subject compared to the reference amount is indicative for a severe form of inflammatory pulmonary disease. Preferably, a decreased amount for the biomarker in the sample of the subject compared to the reference amount is indicative for a mild form of inflammatory pulmonary disease.

Also preferably, said reference amount is derived from a subject or a group of subjects known to suffer from a mild form of inflammatory pulmonary disease and wherein an essentially identical amount or an decreased amount for the biomarker, particularly sFlt1, in the sample of the subject compared to the reference amount is indicative for a mild form of inflammatory pulmonary disease. Preferably, an increased amount for the biomarker in the sample of the subject compared to the reference amount is indicative for a severe form of inflammatory pulmonary disease.

The reference amount applicable for an individual subject may vary depending on various physiological parameters such as age, gender, or subpopulation, as well as on the means used for the determination of the polypeptide or peptide referred to herein. A suitable reference amount may be determined from a reference sample to be analyzed together, i.e. simultaneously or subsequently, with the test sample.

Reference amounts can, in principle, be calculated for a cohort of subjects as specified above based on the average or mean values for a given biomarker by applying standard statistically methods. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity/specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the γ-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/- specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects being at increased risk for mortality or those which have a normal risk among a cohort of subjects suffering from acute inflammation can be generated, preferably, by establishing a ROC for said cohort as described above and deriving a threshold amount therefrom. Dependent on a desired sensitivity and specificity for a diagnostic method, the ROC plot allows deriving suitable thresholds. It will be understood that an optimal sensitivity is desired for excluding a subject for being at increased risk (i.e. a rule out) whereas an optimal specificity is envisaged for a subject to be assessed as being at an increased risk (i.e. a rule in).

Preferably, the median values determined in the Tables of the accompanying Examples may be also used as a basis for establishing thresholds. Preferably, with respect to pneumonia said threshold for sFlt1 which indicates which allows for assessing the severity of inflammatory pulmonary disease, or not, is within the range of the 25^{th} percentile to the 75^{th} percentile of subjects suffering from pneumonia, i.e. about 73 pg/ml to about 206 pg/ml, more preferably it is about the median of 100 pg/ml. Preferably, with respect to COPD said threshold for sFlt1 which indicates which allows for assessing the severity of inflammatory pulmonary disease, or not, is within the range of the 25^{th} percentile to the 75^{th} percentile of subjects suffering from COPD, i.e. about 66 pg/ml to about 106 pg/ml, more preferably it is about the median of about 83 pg/ml.

Moreover, it is particularly preferred that the 95% percentile as determined in clinically healthy individuals may be used for establishing a threshold that serves as a reference amount. Preferably, an individual is considered to be clinically healthy, if said individual does not suffer from inflammatory pulmonary disease. Moreover, the clinically healthy individual shall not suffer from acute inflammation and, thus, shall not suffer from fever, chills or joint paint. In addition, said individual, preferably, has a normal blood pressure, a normal electrocardiogram, no diabetes mellitus and no history of cardiac disease. A particularly preferred threshold for sFlt1 serving as a reference amount is about 88 pg/ml (see Examples).

About in the context of the present invention means +/- 20%, +/- 10%, +/- 5%, +/- 2 % or +/- 1%, or +/- 0% from the said values. This also takes into account usual deviations caused by measurement techniques and the like.

In a preferred embodiment of the aforementioned method, the method further comprises determining the amount(s) of a natriuretic peptide and/ or a cardiac Troponin in a sample from said subject and comparing the determined amount(s) to a reference amount.

The additional determination of a natriuretic peptide and/or of cardiac Troponin further allows for assessing whether the inflammatory pulmonary disease has a cardiac involvement or not. In particular, the additional determination of a natriuretic peptide and/or of cardiac Troponin further allows for assessing whether the inflammatory pulmonary disease is accompanied by heart failure or not. In case of pneumonia, it can be particularly assessed whether the inflammatory pulmonary disease is accompanied by right heart failure or not.

Preferably, an amount of a natriuretic peptide and/or of a cardiac Troponin larger than the reference amount for said natriuretic peptide and/or said cardiac Troponin indicates that the subject also suffers from heart failure.

The term "reference amount" has been described herein above in detail. Preferably, reference amounts for a natriuretic peptide and/or for troponin T are derived from a subject or a group of subjects known to suffer from of inflammatory pulmonary disease and heart failure and wherein an essentially identical amount or an increased amount for the biomarker, particularly a natriuretic peptide and/or a cardiac troponin, in the sample of the subject compared to the reference amount is indicative for inflammatory pulmonary disease accompanied by heart failure. Preferably, a decreased amount for the biomarker in the sample of the subject compared to the reference amount is indicative for inflammatory pulmonary disease not accompanied by heart failure.

Also preferably, said reference amount said reference amount is derived from a subject or a group of subjects known to suffer from inflammatory pulmonary disease not accompanied by heart failure and wherein an essentially identical amount or an decreased amount for the biomarker, particularly natriuretic peptide and/or a cardiac troponin, in the sample of the subject compared to the reference amount is indicative for inflammatory pulmonary disease not accompanied by heart failure. Preferably, an increased amount for the biomarker in the sample of the subject compared to the reference amount is indicative for inflammatory pulmonary disease accompanied by heart failure.

Preferably, a threshold serving as a reference amount for a natriuretic peptide/and or said cardiac Troponin for a subject for a subject suffering from inflammatory pulmonary disease and heart failure is within the range of the 25^{th} percentile to the 50^{th} percentile of a group of subjects suffering from inflammatory pulmonary disease, particularly of a group subjects suffering from a severe form of inflammatory pulmonary disease: In case of pneumonia between 879 pg/ml to 2713 pg/ml for NT-proBNP and between 46 pg/ml and 101 pg/ml for Troponin T. In case of COPD between 130 pg/ml to 506 pg/ml for NT-proBNP and between 4 pg/ml and 13 pg/ml for Troponin T.

With respect to pneumonia, the following applies: Preferred thresholds serving as a reference amount for NT-proBNP are 500 pg/ml, 750 pg/ml 1000 pg/ml. Preferred thresholds serving as a reference amount for Troponin T are 50 pg/ml or 75 pg/ml.

With respect to COPD, the following applies: Preferred thresholds serving as a reference amount for NT-proBNP are 150 pg/ml, 300 pg/ml or 500 pg/ml. Preferred thresholds serving as a reference amount for Troponin T are 10 pg/ml, 20 pg/ml or 30 pg/ml.

In a preferred embodiment of the aforementioned method, the method further comprises recommending a therapy for inflammatory pulmonary disease.

The term "recommending" as used herein means establishing a proposal for a therapy which could be applied to the subject. However, it is to be understood that applying the actual therapy whatsoever is not comprised by the term. The therapy to be recommended depends on the outcome of the diagnosis provided by the method of the present invention.

Preferably, the therapy for inflammatory pulmonary disease is oral or intravenous administration of antibiotics, respiratory therapy, or administration of loop diuretics.

Preferably, if the comparison of the amount of sFlt1 to the reference amount indicates that the subject suffers from a severe form of inflammatory pulmonary disease, the therapy for inflammatory pulmonary disease (and in particular for bacterial pneumonia) is intravenous administration of antibiotics and/or respiratory therapy (if the pO₂ level is low). Moreover, a further therapy for inflammatory pulmonary disease if the amount of sFlt1 in the second sample as compared to the first sample is increased is respiratory therapy, particularly if the pO₂ level is low. Further preferred therapies for severe inflammatory pulmonary disease, particularly for COPD, are administration of bronchodilators such as short-acting β2-agonists, long-acting β2-agonists (preferably, Salmeterol, Formoterol Bambuterol or Indacaterol), or anticholinergics (preferably, ipratropium bromide, oxitropium bromide or tiotropium), oral or intravenous administration of steroids (preferably, prednisone), or administration of theophylline. Preferably, if the comparison of the amount of a natriuretic peptide and/or a cardiac troponin to the reference amount(s) further indicates that the subject suffers from heart failure, the therapy for inflammatory pulmonary disease, preferably, further comprises the administration of loop diuretics. Moreover, the subject may be hospitalized.

Preferably, if the amount of sFlt1 in the second sample as compared to the first sample is increased (and, thus, if the inflammatory pulmonary disease has deteriorated), the therapy for inflammatory pulmonary disease (and in particular for bacterial pneumonia) is intravenous administration of antibiotics. Further preferred therapies for severe inflammatory pulmonary disease, particularly for severe COPD, are administration of bronchodilators such as short-acting β2-agonists, long-acting β2-agonists (preferably, Salmeterol, Formoterol Bambuterol or Indacaterol), or anticholinergics (preferably, ipratropium bromide, oxitropium bromide or tiotropium), oral or intravenous administration of steroids (preferably, prednisone), or administration of theophylline. In addition, if the amount of a cardiac troponin and/or a natriuretic peptide in the second sample as compared to the first sample is increased (and, thus, if the cardiac condition has deteriorated), the therapy for inflammatory pulmonary disease, preferably, further comprises the administration of loop diuretics. Moreover, the subject is, preferably, hospitalized.

Also preferably, if the comparison of the amount of sFlt1 to the reference amount indicates that the subject suffers from a mild form of inflammatory pulmonary disease, the therapy for inflammatory pulmonary disease (and in particular for bacterial pneumonia) is oral administration of antibiotics.

Moreover, the subject suffering from a mild form of inflammatory pulmonary disease, preferably, is not hospitalized or - in case the subject was hospitalized when sample was obtained - discharged from hospital.

In a further preferred embodiment of the method of the present invention, TGFβ-1, BMP2 and/or BMP7 is determined in addition or, preferably, instead of P1GF.

Moreover, the present invention relates to the use of sFlt1 or of a detection agent that specifically binds thereto in a sample of a subject suffering from inflammatory pulmonary disease for monitoring inflammatory pulmonary disease or for assessing the severity of inflammatory pulmonary disease.

Also envisaged by the present invention is the use of i) sFlt1 and at least one further marker selected from a cardiac troponin and a natriuretic peptide or i) of a detection agent that specifically binds thereto and at least one further detection agent that binds to a further marker selected from a cardiac troponin and a natriuretic peptide in a sample of a subject suffering from inflammatory pulmonary disease for monitoring inflammatory pulmonary disease or for assessing the severity of inflammatory pulmonary disease.

The term "detection agent" as used herein refers to an agent which is capable of specifically recognizing and binding to a biomarker present in a sample, preferably to sFlt1, to a natriuretic peptide, or a cardiac Troponin. Moreover, the said agent shall allow for direct or indirect detection of the complex formed by the said agent and the biomarker. Direct detection can be achieved by including into the agent a detectable label. Indirect labelling may be achieved by a further agent which specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker.

Moreover, encompassed is also a device adapted for carrying out the method for monitoring inflammatory pulmonary disease in a patient suffering from inflammatory pulmonary disease comprising
a. an analyzing unit comprising a detection agent which specifically binds to sFlt1 (and preferably, a detection agent which specifically binds to a natriuretic peptide and/or a detection agent which specifically binds to a cardiac Troponin), said unit being adapted for determining the amount of sFlt1 (and preferably, for determining the amount of a natriuretic peptide and/or for determining the amount of a cardiac troponin) in a first and second sample from a subject suffering from inflammatory pulmonary disease; and
b. an evaluation unit for comparing the determined amount in said first sample with the amount in said second sample whereby inflammatory pulmonary disease can be monitored, said unit comprising a database with increases or decreases of the amount of Flt1 (and preferably, of the amount of a natriuretic peptide and/or the amount of a cardiac troponin) in the second sample as compared to the first sample, said increases or decreases being, preferably, derived from a subject or a group of subjects as defined above and a computer-implemented algorithm for carrying out a comparison step.

Moreover, the present invention relates to a device adapted for carrying out the method of the present invention for diagnosing the severity of inflammatory pulmonary disease in a subject suffering from inflammatory pulmonary disease, comprising
a. an analyzing unit comprising a detection agent which specifically binds to sFlt1 (and preferably, a detection agent which specifically binds to a natriuretic peptide and/or a detection agent which specifically binds to a cardiac Troponin), said unit being adapted for determining the amount of sFlt1 (and preferably, for determining the amount of a natriuretic peptide and/or for determining the amount of a cardiac troponin) in a sample of a subject suffering from inflammatory pulmonary disease; and
b. an evaluation unit for comparing the determined amount with a reference amount whereby the severity of inflammatory pulmonary disease can be assessed, said unit comprising a database with reference amount values for sFLT1 (and, preferably, with reference amount values for a natriuretic peptide and/or a cardiac troponin) derived from a subject or group of subjects as defined above and a computer-implemented algorithm for carrying out a comparison step.

The term "device" as used herein relates to a system comprising the aforementioned units operatively linked to each other as to allow the diagnosis or monitoring according to the methods of the invention. Preferred detection agents which can be used for the analyzing unit are disclosed elsewhere herein. The analyzing unit, preferably, comprises said detection agents in immobilized form on a solid support which is to be contacted to the sample comprising the biomarkers the amount of which is to be determined. Moreover, the analyzing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference (e.g. a reference amount, or the amount of the marker in a first or second sample from the subject). Suitable references can be derived from samples of subjects to be used for the generation of reference amounts as described elsewhere herein above. The results may be given as output of parametric diagnostic raw data, preferably, as absolute or relative amounts. It is to be understood that these data will need interpretation by the clinician. However, also envisaged are expert system devices wherein the output comprises processed diagnostic raw data the interpretation of which does not require a specialized clinician.

Moreover, the present invention envisages a kit adapted for carrying out the method of the present invention for monitoring inflammatory pulmonary disease in a subject suffering from inflammatory pulmonary disease comprising a detection agent for the biomarker sFlt1. Preferably, the kit further comprises a detection agent for a cardiac troponin and/or a detection agent for a natriuretic peptide. Moreover, the kit further may comprise instructions for carrying out the said method.

Also, the present invention envisages a kit adapted for carrying out the method of the present invention for diagnosing the severity of inflammatory pulmonary disease in a subject suffering from inflammatory pulmonary disease comprising a detection agent for the biomarker sFlt1. Preferably, the kit further comprises a detection agent for a cardiac troponin and/or a detection agent for a natriuretic peptide. Moreover, the kit further may comprise instructions for carrying out the said method.

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Moreover, the kit may, preferably, comprise standards for reference amounts as described elsewhere herein in detail.

All references cited in this specification are herewith incorporated by reference with respect to their entire disclosure content and the disclosure content specifically mentioned in this specification.

The following examples serve merely as illustration of the invention. They shall not limit the scope of the claims in any way.

### EXAMPLES

### Example 1: Determination of biomarkers sFlt1, NT-proBNP and sensitive troponin T in serum samples

sFlt-1 and NT-proBNP were determined with sandwich immuno-assays using analyzers from Roche/Hitachi, Elecsys or COBAS e-series. The assays comprise two monoclonal antibodies specific for the respective peptide. The first of these iv biotinylated and the second one in labelled with a Tris(2,2'-bibyridyl)ruthemium (II)-complex. In a first incubation step both antibodies are incubated with the sample. A sandwich complex comprising the peptide to be determined and the two different antibodies is formed. In a next incubation step streptavidin-coated beads are added to this complex. The beads bind the sandwich complexes. The reaction mixture is then aspirated into a measuring cell where the beads are magnetically captured on the surface of the electrode. The application of a voltage then induces a chemiluminescent emission from the ruthenium complex which is measured by a photomultiplier. The emitted amount of light is dependent on the amount of sandwich complexes on the electrode. sFlt-1 amounts between 10 to 85,000 pg/ml, and NT-proBNP amounts between 2 pg/ml and 35,000 pg/ml can be measured.

Troponin T (hsTNT) was also determined using the aforementioned automatic analysers. The test followed the same test principles as described for NT-pro BNP. The high sensitivity Troponin T test used in this study has a sensitivity of 1 pg/ml and can be used on ELECSYS 2010 as well as on COBAS e 411 or an COBAS e 601 analysers.

### Example 2: Determination of biomarkers sFlt1, NT-proBNP and sensitive troponin T in 149 clinically healthy individuals

The biomarkers sFlt1, NT-proBNP and sensitive troponin T were determined in 149 clinically healthy individuals (41 males, mean age 40 years range 20 - 52 years; 97 females mean age 41 years (range 18 - 56 years). The clinically healthy individuals had repeatedly normal blood pressure, a normal electrocardiogram, no diabetes mellitus and no history of cardiac disease or other disorders that would have put them at increased risk of cardiac disorders.

Table 1 provides an overview on the results.

**Table 1**

| Percentile | sFlt1 (pg/ml) | NT-proBNP (pg/ml) | sens Troponin T (pg/ml) |
|---|---|---|---|
| 25% | 64.8 | 18.5 | 0.00 |
| 50% | 71.0 | 37.2 | 0.00 |
| 75% | 78 | 67.5 | 0.00 |
| 95% | 88 | 112.0 | 2.9 |

For the studies described in the following the 95 % percentile for sFlt1 (88 pg/ml) was chosen as a threshold for assessing lung disorders

### Example 3: Determination of biomarkers sFlt1, NT-proBNP and sensitive troponin T in patients with stable coronary artery disease

sFlt1, Troponin T and NT-proBNP were determined in 42 patients with stable heart failure, i.e. in patients without cardiac events in the past. In order to exclude cardiac event, the patients were monitored for 4 weeks prior to entry into the study with respect to ECG, echocardiography, clinical signs and symptoms, drug therapy (including compliance of drug therapy, they were not allowed to change drugs and dose). In addition they were not allowed to change body weight for more than 2 kg during the observation period. All patients had a LVEF below 40 %. Samples were obtained before follow up, at 2 weeks, 1 month and 3 months. Data obtained are summarized in the table 2.

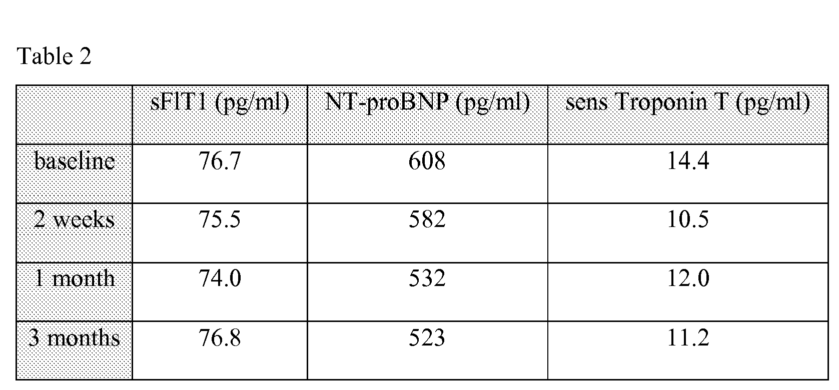

As can be seen from the table 2 above, the median of sFlT1 was stable for the total observation period as was NT-pro BNP and Troponin T. This study confirms the reference value set in healthy subjects as well as the assumption that in stable CAD unrecognized clinical events were unlikely.

### Example 4: Determination of biomarkers sFlt1, NT-proBNP and sensitive troponin T in patients with pneumonia

The biomarkers sFlt1, NT-proBNP and sensitive troponin T were analyzed 123 patients with documented pneumonia, 82 males and 41 females, mean age 62.6 years. Data obtained are summarized in table 3 below:

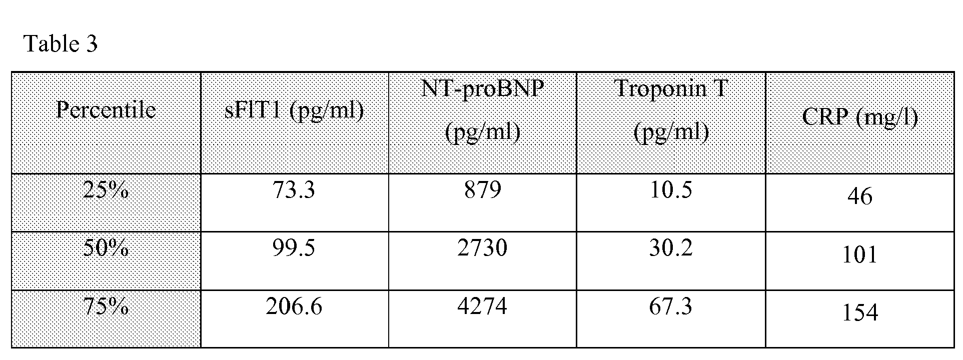

When this group was separated in a group above and below 88 pg/ml sFlT1, the results (median) shown in the following table were obtained:

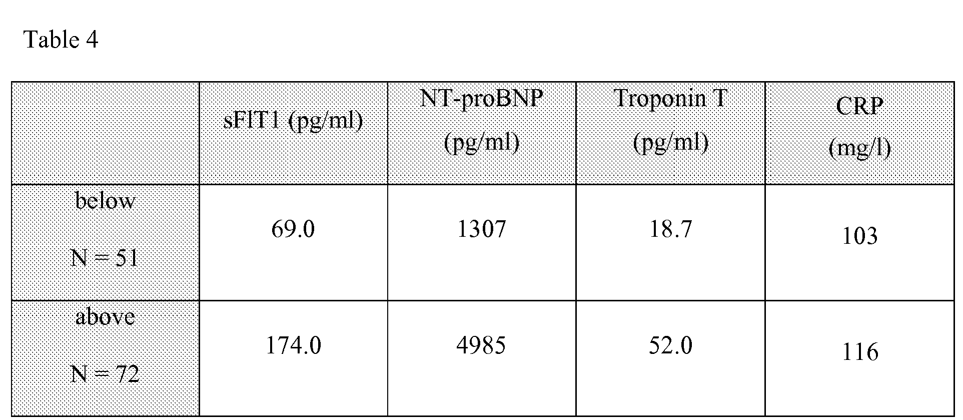

The table indicates that sFlT1 elevation is associated with significantly impaired cardiac function. Interestingly, however, CRP was unrelated to sFlT1 levels and to cardiac function. CRP has been repeatedly shown to increase as early as 4 to 6 hours after an acute inflammatory process (Lobo S.M. et al, Chest 2003, 123 2043 - 2049) and also to be a good predictor of outcome in patients receiving antibiotic therapy (Moreno M.S. J of Infection, 2010 61 205 - 211). However, as shown here, CRP was not associated with inflammatory lung disease as documented by sFlT1 concentration nor with cardiac markers such as NT-pro BNP and Troponin T an thus not useful for the current assessment of pneumonia.

Association of sFlT1 below and above 88 pg/ml respectively we correlated to clinical information:

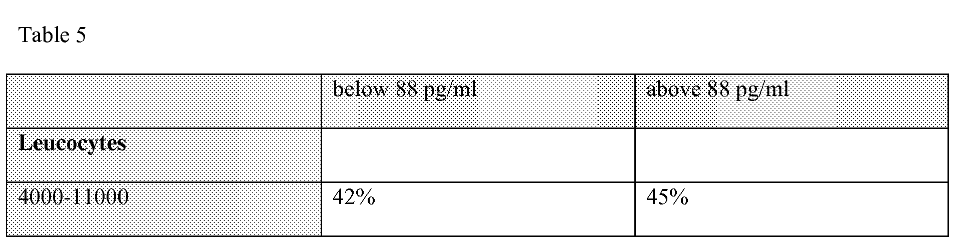

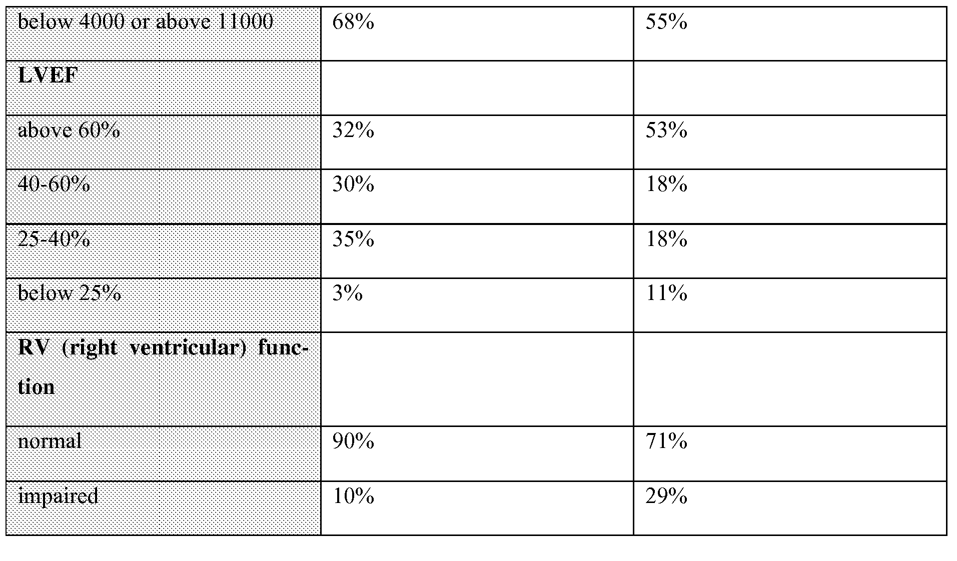

The association of sFlT1 levels above and below 88 pg/ml indicate that both leucocyte count and echocardiography do not significantly add to the assessment. A surprising finding of this study was the lack of association of sFlT1 with abnormal leucocyte counts (below 4000 or above 11000) and left ventricular function (in the presence of highly elevated NT-pro BNP and Troponin T levels in the sFlT1 above 88pg/ml group. sFlT1 was associated with the number of symptoms characteristic of pneumonia and this was independent from imaging results and surprisingly of other laboratory tests used in the assessment of pneumonia such as leucocyte count and CRP level, thus providing additional information to the severity of pneumonia beyond current information. Moreover, there was evidence that impaired right ventricular function was more frequent in this group indicating that elevated sFlT1 was associated (and indicator) of right ventricular dysfunction.

### Example 5: Correlating the sFlt1 level to the severity of pneumonia

Severity of pneumonia can be clinically defined using various parameters of physical examination including altered mental status, respiratory rate of more than 30/min, systolic blood pressure below 90 mmHg, temperature below 35°Celsius or above 40°Celsius and a heart rate of more than 125 beats per minute (Fine M.A. et al NEJM, 1997: 336:243-250). In general, the more of these five criteria are fulfilled, the more severe is the pneumonia. It was analyzed how many of these symptoms were observed in patients with pneumonia with a sFlt1 level below 88pg/ml and a sFlt1 level above 88pg/ml. The results are shown in the following table:

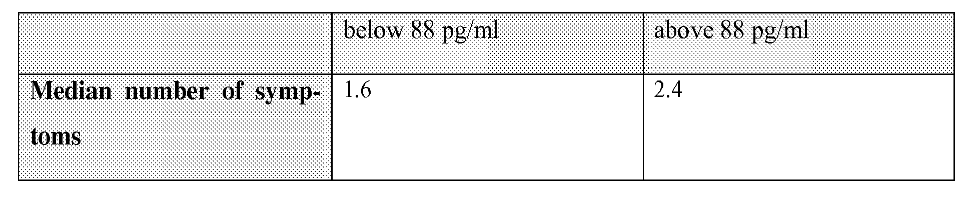

As it can be seen from the table, the median number of symptoms in patients with a sFlt1-level above 88pg/ml is significantly increase as compared to the median number of symptoms in patients with a sFlt1-level below 88pg/ml. Thus, sFlt1 can be used for assessing the severity of pneumonia.

### Example 6: Case Studies (Pneumonia)

The following tables show the levels of sFlt1, NT-proBNP and sensitive troponin T in individuals with pneumonia (baseline: at admission). The individuals belong to the group described above. Individual cases clearly indicate that sFlT1 provides information beyond NT-pro BNP and Troponin T.

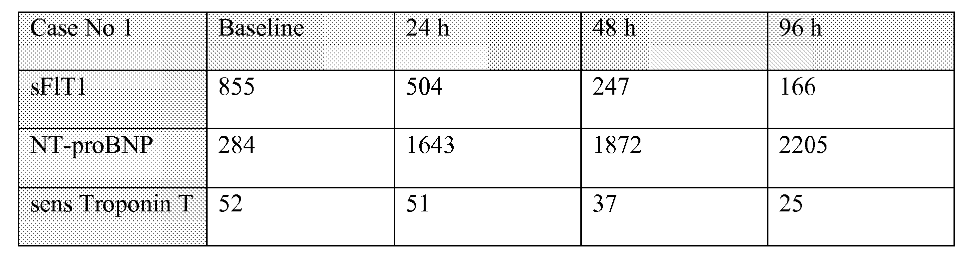

In patient No. 1, the levels of sFlt drops after admission indicating that his condition with respect to pneumonia improved between obtaining the baseline sample and the 96 h sample. This finding was consistent with the clinical observation of a decrease in body temperature from 39,8 degrees celsius to 38,5 degrees celsius, stabilization of the blood pressure with systolic blood pressure consistently above 100 mmHg as well as reduction of the signs of dyspnoe. Thus, the decreasing sFlt1 level indicates that the treatment is effective.

Moreover, a decrease of Troponin T was observed. In contrast, the NT-proBNP levels are increased as compared to the baseline sample. The drop of the sFlt1 and the Troponin T level, however, antecedes the drop of the NT-proBNP level (which is observed after 96 h). This finding is consistent with a decrease in cardiac myocyte death caused by a reduction in ischemia, however this improvement is only followed by improvement of cardiac function with some delay, indicating that both sFlT1 in conjunction with sensitive troponin T can be recognized as early markers for clinical improvement even before definite signs of improvement can be recognized as described above.

Moreover, these findings are consistent with the number of pneumonia specific symptons in patient 1. At baseline, the patient showed four of the five pneumonia specific symptoms as described in Example 5. After 48 hours, the patient only showed two of said symptoms indicating the the condition of the subject has improved.

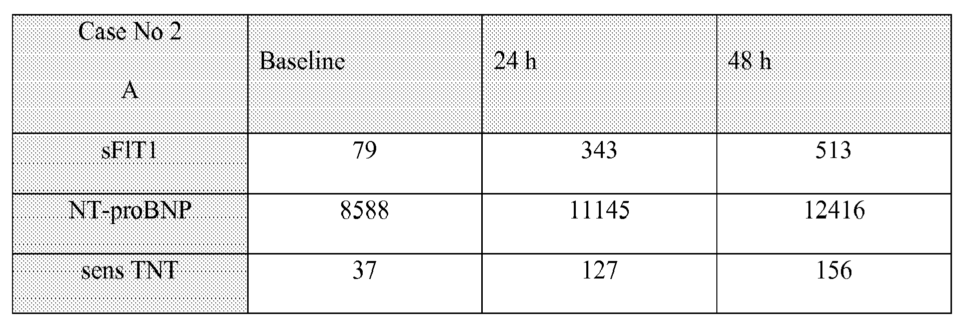

In patient 2, the time course of the level of sFlt1, NT-proBNP and Troponin clearly indicates a deterioration of the condition of the patient. The patient died after the 48 h sample was obtained (approximately 96 h after baseline).

Moreover, these findings are consistent with the number of pneumonia specific symptons in patient 1. At baseline, the patient showed one of the five pneumonia specific symptoms as described in Example 5. 48 hours later, the patient showed three pneumonia specific symptoms indicating the the condition of the subject has deterioated.

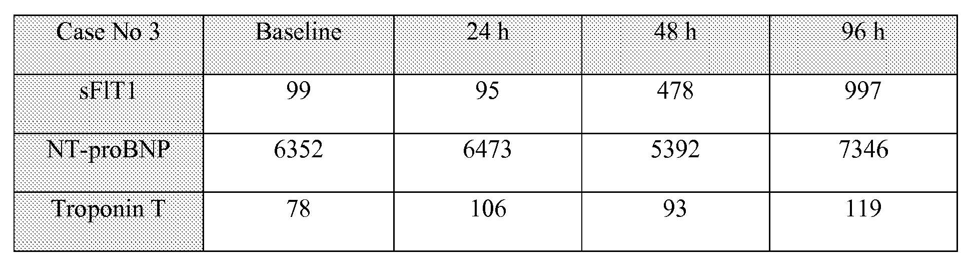

In patient 3, the pneumonia deteriorated after baseline. The cardiac condition also deteriorated slightly. Deterioration of pneumonia was consistent with fever above 40 degrees Celsius, systolic blood pressure of 90 mmHg measured at various times as well as with a respiratory rate of 34/min that did not improve. This finding is also supported by the number of pneumonia specific symptoms. At baseline, the patient show two pneumonia specific symptoms as described in Example 5. However, after 48 hours four pneumonia specific symptoms were observed.

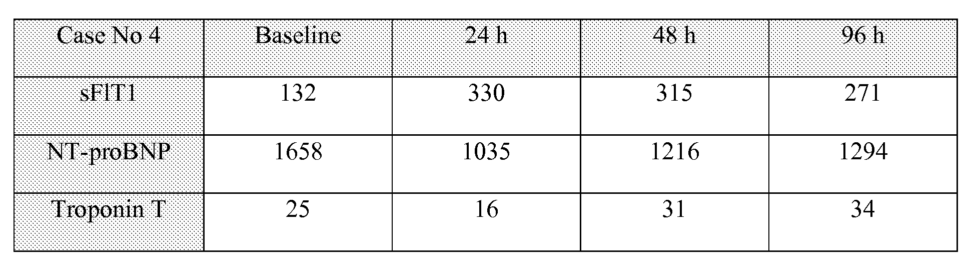

In patient 4, there is an increase of sFlt1 between the baseline sample and the 24 h sample indicating a deterioration of inflammatory lung disease. After 24 h, the sFlt1 level remains stable (48 h) and, then, drops only slightly (96 h). At baseline as well as after 48 hours, the patient showed 3 pneumonia specific symptoms.

### Example 7: Determination of biomarkers sFlt1, NT-proBNP and sensitive troponin T in patients with COPD (chronic obstructive pulmonary disease)

The biomarkers sFlt1, NT-proBNP and sensitive troponin T were determined in 59 patients with COPD (mean age 59.3 years, 41 males and 18 females). The following results were obtained (table 5):

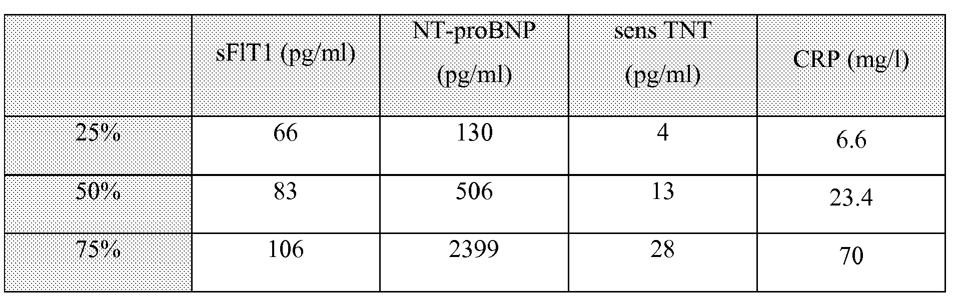

When separated in group below and above sFlT1 88 pg/ml (i.e. the 95^{th} percentile in clinically healthy subjects) the following results were obtained (indicated is the median)

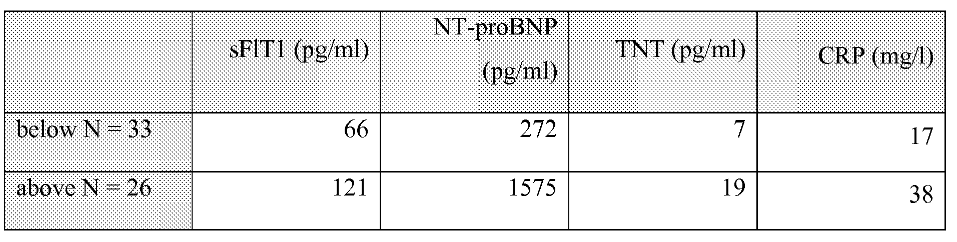

As in pneumonia elevated sFlT1 levels were associated with higher levels of NT-pro BNP and troponin T but also with higher CRP concentrations. Thus, in contrast to pneumonia, sFlt1 was associated with the inflammation marker CRP. This finding is consistent with the fact that exacerbated COPD in contrast to pneumonia (which might be a complication of COPD) does frequently not develop into a systemic infection with cardiac involvement.

The association with clinical or technical information revealed the following results

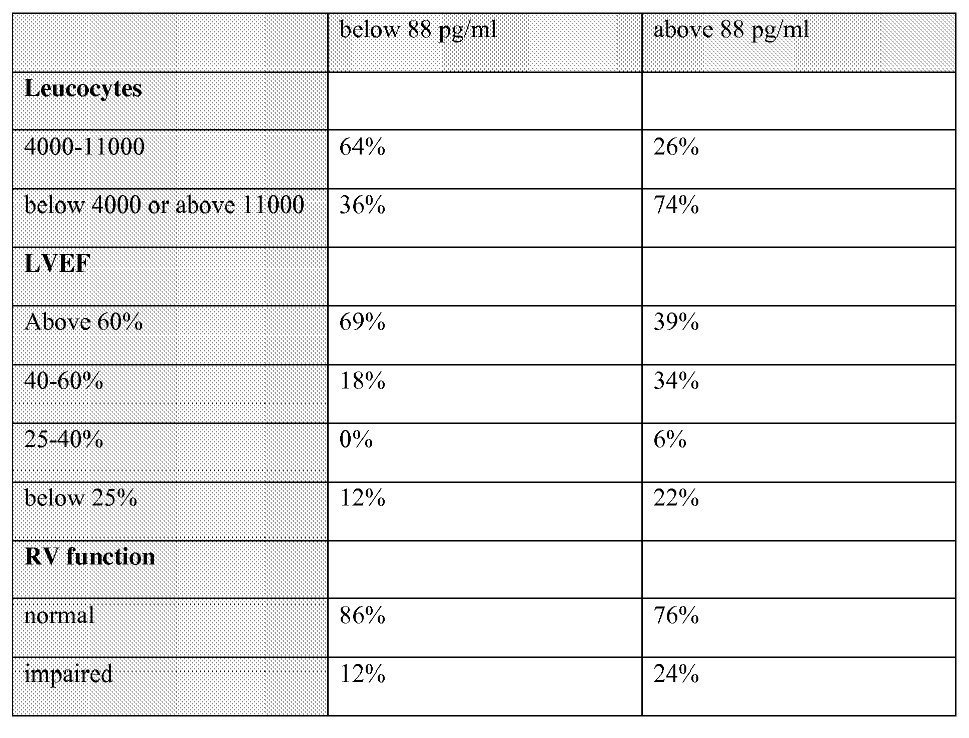

In contrast to pneumonia sFlT1 elevation is associated with technical features such as LVEF and RV function as well as abnormal leucocytes.

### Example 8

A 56 year old patient with known COPD who has smoked for more than 30 years approximately 40 cigarettes per day presents with increasing dyspnoea. His sputum is not purulent. His chest x-ray does not show infiltrates. His sFlT1 is 72 pg/ml. NT-pro BNP is 260 pg/ml and troponin T is 7 pg/ml. No symptoms as described in Example 5 are observed. He is discharged without antibiotics.

### Example 9

A 56 year old patient with known COPD who had smoked for more than 25 years 25 cigarettes per day and who was a miner presents with increasing dyspnoe, fever, chills and purulent sputum. The chest x ray shows basal infiltrates of both lungs, that are reported to reflect pneumonia. His sFlT1 is 215 pg/ml, NT-pro BNP 1830 pg/ml and Troponin T is 52 pg/ml. He shows two of the five clinical symptoms as described in Example 5 and is diagnosed with COPD associated pneumonia and he is kept hospitalised and treated with bron-chodilatators and antibiotics. His fever resolved within 3 days and lung infiltrates were no longer detectable after 1 week and antibiotic treatment was discontinued. Control blood testing revealed sFlT1 of 91 pg/ml, Troponin T was 12 pg/ml and NT-pro BNP 415 pg/ml. The patient was discharged and increased Troponin T and NT-pro BNP values at presentation are considered as cardiac involvement of a bacterial systemic infection. The patient is recommended to have a further follow up in 2 weeks.

### Example 10

A 56 year old female with metastatic beast cancer on cancer therapy presented with fever. chills, dyspnoe and leucocyte counts of 1450/µl at the emergency department. Respiratory rate is 32/min and her blood pressure is 90/75. The patient does not have a history of heart failure or coronary artery disease. Her sFlT1 is 189 pg/ml. NT-proBNP is 3950 pg/ml and Troponin T is 53 pg/ml. Chest x ray revealed pulmonary infiltrates compatible with pneumonia, she is given 02 4 1/min and intravenous antibiotics. She has an uneventful course, her leucocytes increased to 5200/µl, sFlT1 is 69 pg/ml after 10 days, NT-pro BNP 215 pg/ml and Troponin T 3 pg/ml. She is diagnosed with cardiac involvement in the context of a systemic infection and discharged, infiltrates has disappeared on control chest X ray.

### The data presented herein provide the following diagnostic information:

sFlt1 is associated with the severity of inflammatory pulmonary disease. Thus, when using suitable control levels such as from control groups (normal subjects and/or subject with stable cardiovascular disease and concomitant stable heart failure and/or subjects with inflammatory lung disease) the severity of pneumonia can be assessed. Moreover, the marker allows for assessing the severity of inflammatory lung disease. The aforementioned diagnostic methods, thus, allow for monitoring of inflammatory pulmonary disease and for assessing the severity of inflammatory lung disease. Based on this differentiation, therapeutic needs can be determined, such as, e.g., a need for intravenous administration of antibiotics, and suitable therapies can be immediately applied.

## Claims

1. A method for monitoring inflammatory pulmonary disease in a patient suffering from inflammatory pulmonary disease, said method comprising the steps of
a. determining the amount of soluble fms-like tyrosine kinase 1 (sFlt1) in a first sample of said a subject,
b. determining the amount of sFlt1 in a second sample of said subject obtained after said first sample; and
c. comparing the amount of sFlt1 in said first sample to the amount of sFlt1 in said second sample.

2. The method of claim 1, wherein a decrease of the amount in said second sample as compared to said first sample indicates amelioration of said inflammatory pulmonary disease, wherein an increase of the amount in said second sample as compared to said first sample indicates deterioration of said inflammatory pulmonary disease, and/or wherein an essentially unchanged amount in said second sample as compared to said first sample indicates no change in status of said inflammatory pulmonary disease.

3. The method of claim 1 and 2, wherein said inflammatory pulmonary disease is pneumonia or chronic obstructive lung disorder (COPD).

4. The method of any one of claims 1 to 3, wherein said sample is a blood, serum or plasma sample.

5. A method for diagnosing the severity of inflammatory pulmonary disease in a subject suffering from inflammatory pulmonary disease, said method comprising the steps of
a. determining the amount of soluble fms-like tyrosine kinase 1 (sFlt1) in a sample of said a subject,
b. comparing said amount of sFlt1 to a reference amount, whereby the severity of inflammatory pulmonary disease is diagnosed.

6. The method of claim 5, wherein the subject has a history of heart failure.

7. The method of claims 5 and 6, wherein said reference amount is derived from a subject or a group of subjects known to suffer from a severe form of inflammatory pulmonary disease.

8. The method of claim 7, wherein an essentially identical amount or an increased amount for the biomarker in the sample of the subject compared to the reference amount is indicative for a severe form of inflammatory pulmonary disease.

9. The method of any of claims 5 to 8, further comprising determining the amount(s) of a natriuretic peptide and/ or a cardiac Troponin in a sample from said subject and comparing the determined amount(s) to a reference amount.

10. The method of any of claims 1 to 9, further comprising recommending a therapy for inflammatory pulmonary disease.

11. The method of claim 10, wherein the therapy for inflammatory pulmonary disease is oral or intravenous administration of antibiotics, respiratory therapy, administration of loop diuretics.

12. The method of any one of claims 1 to 11, wherein the amount TGFβ-1 is determined instead of the amount of sFlt1.

13. Use of sFlt1 or of a detection agent that specifically binds thereto in a sample of a subject suffering from inflammatory pulmonary disease for monitoring inflammatory pulmonary disease or for diagnosing the severity of inflammatory pulmonary disease.

14. A device adapted for carrying out the method of any one of claims 5 to 9 comprising
a. an analyzing unit comprising a detection agent which specifically binds to sFlt1, said unit being adapted for determining the amount of sFlt1 in a sample of a subject suffering from inflammatory pulmonary disease; and
b. an evaluation unit for comparing the determined amount with a reference amount whereby the severity of inflammatory lung can be assessed, said unit comprising a database with reference amount values derived from a subject as set forth in claim 7 and a computer-implemented algorithm for carrying out a comparison step.

15. A kit adapted for carrying out the method of any one of claims 1 to 9 comprising a detection agent for the biomarker sFlt1.
